# EUROPEAN PATENT SPECIFICATION

(11) **EP 3 429 555 B1**
(45) Date of publication and mention of the grant of the patent: **29.04.2020**
(21) Application number: 17707572.8
(22) Date of filing: 01.03.2017
(51) Int. Cl.: A61K 9/08, A61K 47/02, A61K 9/19, A61L 31/16, A61B 17/3203, A61L 2/00, A61K 31/185

(54) **MESNA FORMULATION AND ITS USE**
MESNA-FORMULIERUNG UND VERWENDUNG DAVON
FORMULATION DE MESNA ET SON UTILISATION

(30) Priority: 14.03.2016 EP 16160165; 18.10.2016 BE 201605775
(43) Date of publication of application: 23.01.2019
(73) Proprietor: AuXin Surgery SA, 1348 Louvain La Neuve (BE)
(72) Inventor: CAPART, Gilles, 1200 Brussels (BE); VERJANS, Benoit, 1050 Brussels (BE)
(74) Representative: Brantsandpatents bvba
(86) International application number: PCT/EP2017/054829
(87) International publication number: WO 2017/157670

(56) References cited:
- WO-A1-2014/180902
- WO-A1-2015/101665
- US-A- 5 728 738
- US-A1- 2005 124 589
- US-A1- 2015 141 412

## Description

### Field of the invention

The present invention relates to a device for delivering Sodium 2-mercaptoethanesulfonate (Mesna) formulation to tissues and/or organs. The invention further provides the formulation and a process for its preparation. The formulation and/or the device and/or the process can be used for chemical assisted surgery.

### Background

It is known that when applied at the cleavage plane, sodium-2-mercaptoethane sulfonate or Mesna breaks the disulfide bonds between tissue layers, thereby facilitating tissue separation. Specifically, Mesna breaks disulfide bonds of polypeptide chains and proteins. However, nowadays for tissue separation, Mesna is only commercially available in pharmaceutical dosage forms intended for other applications. A major drawback of Mesna solutions is their instability as mentioned in US5728738. The liquid form is highly prone to oxidation and is therefore highly unstable especially in presence of catalyzing metal ions. Therefore, it is common to store Mesna solutions in low iron glass containers under nitrogen blanket with stabilizers, ion chelating agents and anti-oxidants. When being used, the practitioner has to transfer the solution from glass containers to a delivery device or to a tube in order to bring the solution in contact with the desired tissue. This step is not convenient, increases contamination risk, for instance by bacteria and/or by glass particles, at the dissection site and increases surgery time. This is in addition to the high chances of oxidation of the stored Mesna solution thereby having a reduced Mesna activity when used for assisting surgery.

Other drawback of Mesna in liquid form available up to date resides in the absence of concentration choice. In some procedures, larger quantities and/or different concentration of Mesna, from that readily available in commercial glass containers, are needed. Indeed the practitioner can only dilute the Mesna which is available in liquid form and cannot use higher Mesna concentrations if required. This makes the use of said containers tedious and inadequate. As mentioned in US5728738, Mesna solutions available are stabilized using pH adjustment agents and/or additives such as antioxidants and stabilizing agents thereby avoiding oxidation and/or the degradation of Mesna when the Mesna solution is stored. The use of such agents presents a significant risk for patient's health (authorities recommend to avoid such products in injectable solutions) and increases the cost of said Mesna solutions. In addition, commercial solutions are strongly hypertonic and may damage exposed cells when applied topically.

The object of the invention is to overcome at least part of the above mentioned problems. One of the objectives of the invention is to provide a Mesna formulation having a pH which is close to physiological pH and/or closer to isotonicity. Another object of the invention is to provide a process for preparing such formulation and a device for preparing and delivering said formulation to tissues and/or organs. These object and other objects are achieved in accordance with the invention by a formulation, a process and devices as described below and in the appended claims.

### Summary

The present invention provides a device for delivering a Mesna formulation to tissues and/or organs, comprising a first chamber comprising Mesna, a second chamber comprising a buffer and at least one outlet for delivering the Mesna formulation, said outlet is in fluid communication with at least one of the chambers; said chambers are separated from each other by at least one disruptable separation means and are in fluid communication with each other upon disruption of said separation means thereby forming the Mesna formulation, characterized in that the pH of the buffer comprised in the second chamber is at least 8.5.

In another aspect, the invention provides a process for the preparation of a Mesna formulation, comprising the steps of dissolving Mesna in a buffer thereby obtaining the Mesna formulation, wherein the process is devoid of steps in which stabilizers and/or antioxidants are added after dissolving Mesna in the buffer and is characterized in that the buffer pH is at least 8.5. Preferably, the Mesna solution is prepared within a device according to an embodiment of the invention.

The invention further provides a Mesna liquid formulation comprising Mesna which is dissolved in a buffer having a pH of at least 8.5. Said formulation is prepared immediately prior use and has a pH of from 6 to 8. The Mesna concentration of the formulation at most 10%, even more preferably at most 8%, most preferably at most 5%. The liquid formulation is sterile and has a pH of from 6 to 8. The formulation is devoid of anti-oxidants and/or stabilizers.

The invention additionally provides a process for preparing a liquid Mesna formulation. Preferably, said liquid Mesna formulation is as described above. The process comprises the step of dissolving Mesna in a buffer having a pH of at least 8.5, preferably at least 8.7, more preferably at least 8.8, most preferably at least 9. Said pH is the pH of the buffer before reconstitution, so before dissolving Mesna in the buffer. The process is characterized in that it is devoid of steps in which stabilizers and/or antioxidants are added after dissolving Mesna in the buffer.

In another aspect, the invention provides a kit comprising a device according to an embodiment of the invention, optionally a first container comprising Mesna in solid form and a second container comprising a buffer having a pH of at least 8.5 and a leaflet comprising instructions to the user. The kit optionally comprises at least one tubing and a system enabling the surgeon to control dispensing during surgery.

The device of the invention is suitable to be used as or as an integral part of a chemically assisted tissue dissection system. Said device is suitable to be connected to any surgical dissector known to the person skilled in the art provided with fluid connection for dispensing at the active edge of said surgical dissector. The device allows the topical and local instillation of a chemical solution to facilitate the mechanical dissection and separation of tissue.

The device enables the controlled instillation of Mesna during tissue dissection directly in contact with the tissue to be detached thus reducing its diffusion. Mesna facilitates the mechanical tissue separation by the surgical dissector. The chemical compound for instillation is located in the device and thus forms an integral part of the device. Mesna is stored in the device in solid form before use to take advantage of the superior stability properties of the solid form compared to the formulated solution.

The formulation, the process and the device of the invention present several advantages. The formulation is devoid of antioxidants and/or stabilizing agents, the use of which is not required as the solution is prepared immediately prior use. This reduces the risk of potential side effects from the use of such additives, ensures high Mesna activity and reduces the costs of the solution. The invention further provides a maximized sterility of the delivered Mesna solution and thereby of the surgery and/or the treatment. In addition, the invention provides for a controlled delivery of the Mesna solution which can be delivered at the practitioner's will and at the required time. It avoids cumbersome manipulations of solutions from glass containers in order to obtain the desired concentration and volume. Furthermore, the invention offers to the device manufacturer the possibility to supply multiple concentrations of Mesna solution to be used by the practitioners thanks to various ratios of Mesna and buffer quantities.

The device containing the Mesna formulation according to the invention is easy to use and facilitates tissue dissection while preserving healthy tissue and organ functions. Observable benefits are to reduce the damage to the remaining tissues or organs (including surrounding tissues and organs), a reduction of pre- and post-operative bleeding, a reduction of post-operation adhesions, a reduction in surgical procedure time, a reduction of surgical complications and an increased surgeon satisfaction. In addition, the invention allows decreasing hospital stay duration, preventing post-operative complications and allows decrease disease recurrence.

The device and/or the process and/or the method according to any embodiment of the present invention, allows mixing Mesna in solid form with a buffer having a pH of at least 8.5 shortly prior use. This provides a Mesna formulation having an acceptable pH for use in surgery and provides a maximum Mesna activity as oxidation of said Mesna is reduced compared to Mesna formulations stored for a long time prior use. The Mesna formulation of the invention is prepared and/or delivered to the target at most 24 hours, preferably at most 12 hours, more preferably at most 8 hours, even more preferably at most 4 hours, most preferably at most 30 min, even most preferably at most 10 min after dissolving the solid Mesna in the buffer.

### Brief description of the figures

**Fig. 1** shows a first embodiment of the device according to the invention.

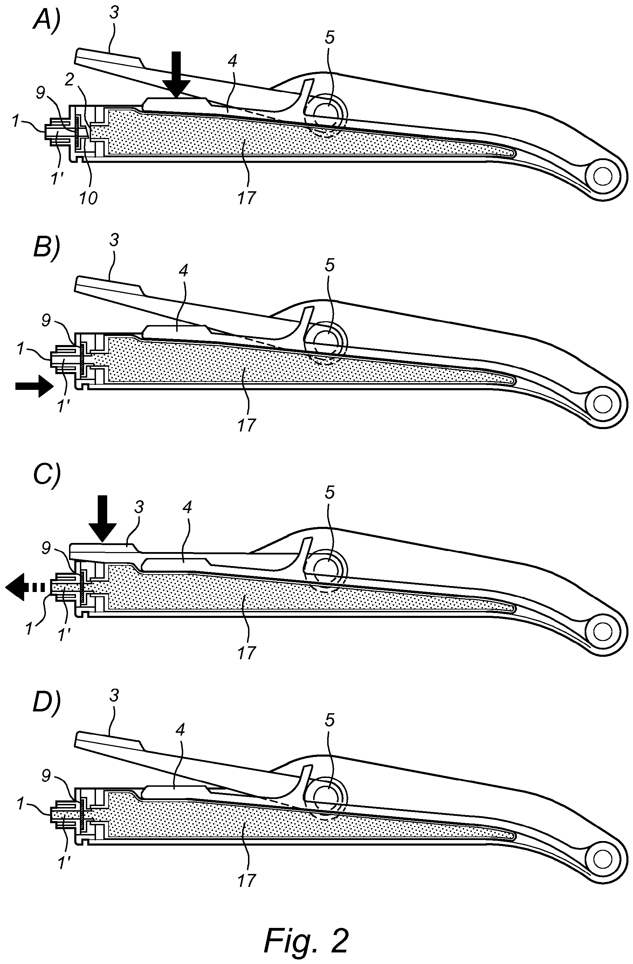

**Fig. 2** **A to D** shows the steps of use of the device presented in **Fig. 1****.**
**Fig. 3** shows a second embodiment of the device according to the invention.
**Fig. 4** **A to D** shows the steps of use of the device presented in **Fig. 3****.**
**Fig. 5A** shows a perspective view of a device according to a third embodiment of the invention.
**Fig. 5B** shows a cross section view of the device of **Fig. 5A** wherein the chambers are not pierced.
**Fig. 5C** shows a cross section view of the device of **Fig. 5A** wherein the chambers are pierced.

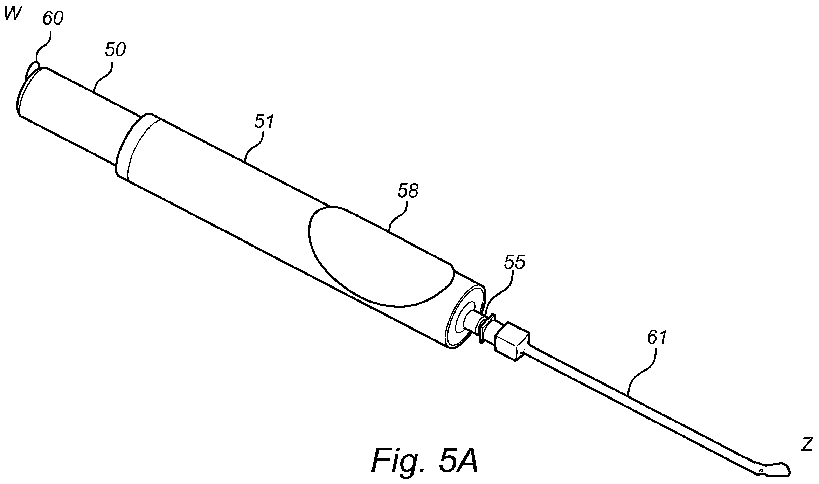

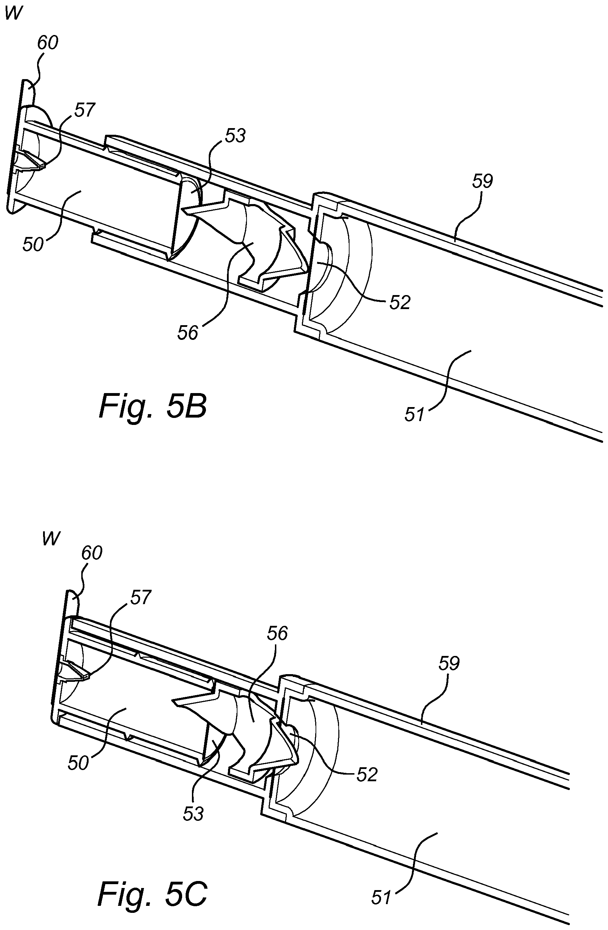

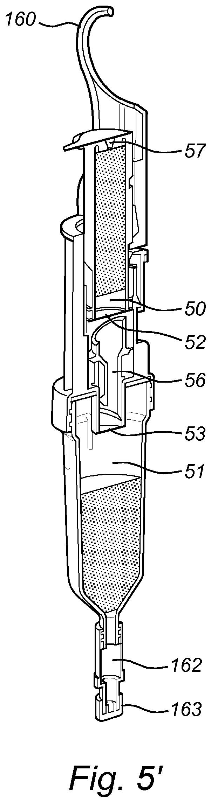

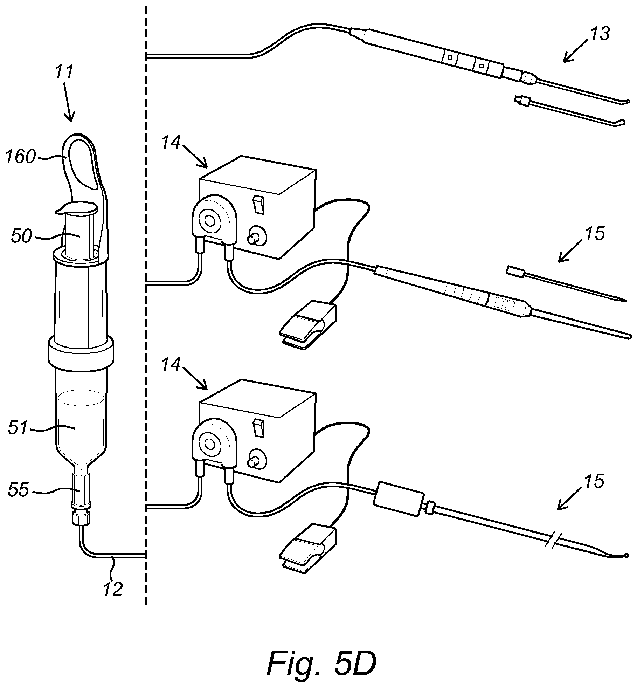

**Fig. 5'** shows a longitudinal cross section of the device shown in **Fig. 5A**
**Fig. 5D** shows the different uses and possible connections of an embodiment of the device of **Fig. 5A****.**

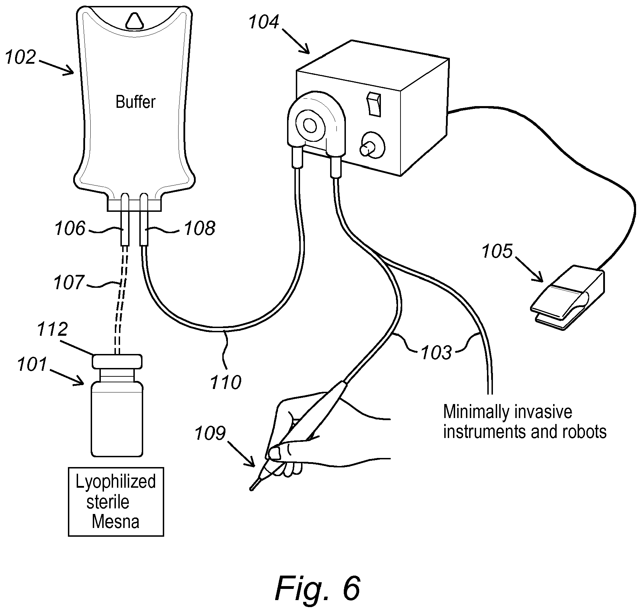

**Fig. 6** shows another embodiment of the device according to the invention.

### Detailed description

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% (Mesna) weight/volume", here and throughout the description unless otherwise defined, refers to the relative weight of the respective solid component versus the weight of the solution, prior to mixing of both components, assuming this solution has a density of 1 gr/ml.

"Mesna formulation" and "Mesna solution" are herein used as synonyms and refer to the aqueous solution obtained after dissolving Mesna is solid form in the buffer as provided by the invention.

WO 2014/180902 A1 discloses a device for delivering a solution to tissues and/or organs, the solution comprising at least one solvent and at least one solute. Said device of WO 2014/180902 A1 comprises at least one chamber which comprises sodium 2-mercaptoethanesulfonate in powder form. WO 2014/180902 A1 further discloses a method comprising the steps of dissolving the solute in the solvent inside the device thereby obtaining the solution, and delivering immediately the obtained solution to said tissues and/or organs to facilitate dissection.

US 2005/124589 A1 concerns the use of mercaptoethane sulfonate-sodium (Mesna) to increase the solubility of Ifosfamide in storage-stable, concentrated and/or highly-concentrated (supersaturated) aqueous pharmaceutical preparations, storage-stable, concentrated and/or highly-concentrated (supersaturated) aqueous pharmaceutical Ifosfamide preparations for parenteral administration as well as a process for their production.

The present invention provides a device for preparing and/or delivering a Mesna formulation to tissues and/or organs. The device comprises a first chamber and a second chamber. The first chamber contains Mesna. Said Mesna is preferably in solid form. The second chamber comprises a solvent. The device further comprises at least one outlet for delivering the formulation; said outlet is in fluid communication with at least one of the chambers. The chambers are separated from each other by at least one disruptable separation means and are in fluid communication with each other upon disruption of said separation means thereby forming the Mesna formulation. The pH of the buffer comprised in the second chamber is at least 8.5, preferably at least 8.7, more preferably at least 8.8, most preferably at least 9.

The device comprising the chambers might be provided in a packaging or a pouch before terminal sterilization by gamma, X-Ray or Beta irradiation. An oxygen scavenger is preferably placed in the pouch before sealing it to increase the shelf life of the device and/or to prevent eventual Mesna oxidation from oxygen in presence of water vapor crossing the walls of the liquid container. Immediately before use, the device is removed from the packaging. The user activates the disruption means thereby obtaining the Mesna formulation which can be used in surgery.

The inventors surprisingly found that Mesna either in crystalline pure API form or in lyophilized form can be sterilized by gamma, X-Ray and Beta irradiation. The irradiation is preferably performed at least at 20 kGy, preferably at least 25 kGy, more preferably at least 30 kGy and at most 50 kGy, preferably at most 45 kGy. The lyophilized form can be sterilized by ultrafiltration in solution before the lyophilization step.

The buffer might be obtained by mixing a solvent with at least one buffer excipient. The solvent might consist of water of high purity (e.g. water for injection as per pharmaceutical standard) and may contain between 0 and 0.9% of NaCl. The pH of the buffer might be adjusted using pH adjustment agents to reach the desired pH of at least 8.5. The pH of the buffer allows obtaining a Mesna formulation having a pH of from 6 to 8 by only adding solid Mesna in powder form or in lyophilized form to the buffer and without addition of pH adjustment agents. This avoids manipulation of the obtained Mesna formulation, to adjust the pH for instance, thereby providing for the immediate use of the obtained liquid Mesna formulation. This ensures a maximal sterility of the formulation and a maximal safety for patient thanks to the use of a solution with a pH close to physiological pH.

The buffer may further contain between 0 and 0.9% of NaCl as a source of chlorine ions. This reduces the side-effects due to chlorine ion depletion. In particular, lack of sufficient chlorine ions may trigger unexpected hyperactivity of some cells such as neurons in brain and in the nerves.

The buffer comprises phosphate such that the phosphate to Mesna ratio of the Mesna formulation is at least 1/500, preferably at least 1/400, more preferably at least 1/300, even more preferably at least 1/250, most preferably at least 1/150, even most preferably at least 1/100. Said ratio is at most 1/1, preferably at most 1/1.5, more preferably at most 1/2, most preferably 1/3. Preferably, the buffer comprises or consists of Na₂HPO₄ and NaCl. More preferably, the buffer comprises or consists of 10 mM Na₂HPO₄ and 75 mM NaCl. The buffer can also comprise or consist of Na₂HPO₄, more preferably, 10 mM Na₂HPO₄.

The buffer excipient is selected from the group comprising sodium acetate, acetic acid, glacial acetic acid, ammonium acetate, arginine, aspartic acid, benzoate sodium, benzoate acid, carbonate sodium, bicarbonate sodium, citrate acid, citrate sodium, citrate disodium, citrate trisodium, glucono delta lactone, glycine, glycine HCI, histidine, histidine HCI, hydrobromic acid, meglumine, phosphate acid, phosphate monobasic potassium, phosphate dibasic potassium, phosphate monobasic sodium, phosphate dibasic sodium, tartrate sodium, tartrate acid, tromethamine (tris) or any combination thereof. The concentration of the buffer excipient is of from 2 mM to 60 mM, preferably from 2.5 mM to 50 mM, more preferably from 5 to 40 mM, more preferably from 7.5 mM to 35 mM, most preferably from 10 mM to 30 mM.

The solid Mesna might be Mesna in powder form, also herein called crystalline form or pure crystalline Mesna from API production. Solid Mesna might also be in lyophilized form. The Mesna from API production preferably meets the pharmacopoeia criteria. The lyophilized form is preferably obtained by lyophilizing a Mesna solution resulting from dissolving Mesna powder in pure water for injection. The lyophilized form is preferably as described in patent WO2015/101665 which content is included herein. Said Mesna solution might be sterilized, preferably by micro filtration prior to lyophilization. Solid Mesna is stable under normal conditions and eliminates the need for special precautions to prevent oxidation of Mesna solutions during storage. Normal conditions refer to room temperature and normal humidity level.

The final Mesna concentration in the obtained formulation at most 10%, even more preferably at most 8%, most preferably at most 5%. Said Mesna concentration is at least 1%, preferably at least 2%, more preferably at least 3% and most preferably at least 4% or any value comprised between the aforementioned values. These concentrations are advantageous for topical applications, such as in surgery, to limit Mesna cytotoxicity while maintaining sufficient Mesna activity. The hypertonic characteristic of formulations having Mesna concentrations of 10% or higher is a concern in topical applications. The experiments show that the toxicity on cells and organs is a consequence of the hypertonic character of Mesna formulations having a concentrations of 10% or higher. Formulations having Mesna concentrations of 5% or less show reduced and almost absent toxicity. Said toxicity is not a consequence of using Mesna as such. In addition, the pre-clinical and clinical studies in surgery show effectiveness of all Mesna concentrations on disulfide bonds cleavage.

Preferably, the pH of the obtained formulation is of from 6 to 8, preferably from 6.8 to 7.8, more preferably around 7.3. Said formulation pH is obtained without pH adjustments, i.e. without use of pH adjustment agents or molecules. It is to be understood that the pH of the buffer itself - so before dissolving solid Mesna in said buffer - might be adjusted using pH adjustment agents to have a buffer with a pH of at least 8.5, preferably at least 8.7, more preferably at least 8.8, most preferably at least 9. The pH of the Mesna formulation - so after mixing Mesna with the buffer - is not adjusted with pH adjustment agents and is of from 6 to 8. High pH values of the buffer, i.e. at least 8.5, preferably at least 8.7, more preferably at least 8.8, most preferably at least 9, reduces the risk of bacterial growth and avoids the use of stabilizers.

The buffer is preferably devoid of carbon atoms. The lack of carbon inhibits and/or prevents bacterial growth thereby enhancing the microbiological quality of the buffer and the Mesna formulation. In particular, the lack of carbon ensures low level of bioburden within the buffer and the Mesna formulation. The lack of carbon further ensures limited or no endotoxin contamination of the buffer and thereby of the Mesna solution. Additionally, the absence of carbon atoms does not provide additional tonicity to the buffer and/or to the Mesna formulation.

The separation means might comprise a spatial separation between the first chamber and the second chamber. This means that when the device is not in use, the chambers do not share any common element, such as membranes and/or walls. In this embodiment, before using the device, a fluid connection is established between the two chambers in order to reconstitute the Mesna solution in the second chamber. The second chamber is then connected to the user applications, such as surgery. Preferably, when the device is used, at least one of the first or second chambers is movable towards the other chamber and/or both chambers are movable towards each other. One of the advantages of this design is that it offers the possibility to fill the chambers separately from each other in a sterile/clean environment. Assembling the device can then later be performed in a non-sterile environment. After assembling the device, a terminal sterilization step can be performed.

The separation means might be devoid of spatial separation between the first chamber and the second chamber. For instance, the first chamber and the second chamber can be attached to each other by said separation means which separates the chambers from each other. After removal of the separation means immediately prior use, the chambers are in fluid communication with each other and the reconstituted solution can be dispensed to the user.

Preferably, the separation means comprises at least one disruption means for disrupting said separation means. Preferably, the device further comprises a disruptable sealing membrane positioned between the outlet and the chamber which is suitable to be in fluid communication with said outlet.

Preferably, the device comprises at least one control means which provides for the control of the solution volume flowing out of the device. Said control means might be any means known to the person skilled in the art

The device might comprise at least one sterilization microfilter positioned between the outlet of the device and the chamber which is suitable to be in fluid communication with said outlet. Preferably said microfilter is a membrane made of polyethylene terephtalate, polyamide, polyethersulfone, nylon or any other suitable material. In a preferred embodiment, the pore size is from 0.1 to 3 µm, preferably from 0.15 to 2 µm, more preferably from 0.2 to 1 µm, most preferably about 0.22 µm. The presence of said microfilter further enhances the sterility of the solution and/or ensures the instillation of the sterile solution of the device, i.e. Mesna solution, during dissection. In a preferred embodiment, said microfilter has an additional ventilation membrane to remove air bubbles during dispensing. In a preferred embodiment, the pore size of the ventilation membrane is from 0,01 to 0,05 µm, preferably about 0,02 µm. The ventilation membrane is preferably made of polytetrafluoroethylene or any other suitable material.

The device might comprise at least one particle filter positioned between the chambers of the device. Said particle filter eliminates accidental particles that might be released during the dissolution of Mesna in solid form in the buffer. The pore size of the particles filter is from 0.1 to 10 µm, preferably from 0.22 to 8 µm, more preferably from 0.5 to 6 µm, most preferably 1 to 5 µm.

The outlet of the device is connectable to at least one second device selected from the group comprising surgical devices, high pressure pumps, delivery tubes, applicators, minimally invasive surgery systems, robot assisted device and low pressure pump. The outlet of the device is connectable to high pressure pumps for use in hydrosurgery. Said outlet might be connected via a luer lock mechanism to said second device. The surgical device can be a dissector instrument, known to the person skilled in the art, which primary function is tissues separation in surgery through mechanical action. Said dissector is a general manual surgical instrument used in gynecology, ENT surgery, orthopedics, neurosurgical and all other surgical procedures where tissues need to be separated. Said dissectors are stainless steel or titanium instruments with different hooks and curves, such as long- medium, short beveled hook, hook with ball, straight and curved, big and small size versions. The dissectors may be fitted with internal capillary tube for dispensing the fluid at their active edge. The shape of the dissector to be used in surgery depends on the application and area. Many dissectors are re-usable or disposable, in plastic, stainless steel, titanium or other metals. The dissector can also be fitted with a cavity and a second capillary tube for the suction of the excess liquids and Mesna solution, as commonly used for removing excess liquids from the operating field during the procedure that would otherwise prevent complete vision of the field. In a preferred embodiment, said surgical device or delivery tube can be combined with suction and/or suction/irrigation devices used in open and minimally invasive surgery. For minimally invasive surgery, said surgical device or delivery tube can be inserted in the instrument channel of suction/irrigation devices.

The device, according to any embodiment of the invention, can be connectable to and/or controllable by an electrically driven mechanical system for the delivery of Mesna solution to the target location. Said system can be a syringe driver, a syringe pump or any other system known to the person skilled in the art. Said system is preferably controlled by the practitioner using a pedal connected to the electrically driven mechanical system. This is advantageous as it provides the practitioner with a high hands freedom level required for instance for laparoscopic surgery also called minimal invasive surgery. Indeed, said practitioner will be able to use both hands for operations other than handling the device for delivering Mesna solution to the target location.

The device, according to any embodiment of the invention, can also be hand held and hand manipulated by the operator. Where required, the device is further provided with a plunger which is used by the operator for delivering said Mesna solution. Said plunger is slidably engaged into one chamber of the device.

The device is a single-use device. Preferably, it is a single-use chemically assisted tissue dissector indicated for the cleavage and separation of tissue layers to facilitate various surgical procedures, including abdominal surgery, thoracic surgery, urology, gynecology, orthopedics and otoneurosurgery.

The device and/or the process according to any embodiment of the present invention, allows mixing Mesna in solid form with a buffer having a pH of at least 8.5 shortly prior use. The obtained Mesna solution does not require any pH adjustment and can be used immediately. The use of the obtained solution provides for a maximum Mesna activity as Mesna oxidation is considerably reduced compared to Mesna solutions stored for a long time prior use. The Mesna solution is prepared and/or delivered to the target at most 24 hours, preferably at most 12 hours, more preferably at most 6 hours, even more preferably at most 4 hours, most preferably at most 30 min, even most preferably at most 10 min after dissolving the solute in the solvent. In a preferred embodiment, the device is designed to contain 5 to 200 ml, preferably 10 to 150 ml, more preferably 15 to 100 ml, most preferably 20 to 50 ml of buffer or any volume comprised within the mentioned ranges. Preferably, the device is designed to contain about 30 ml of buffer.

The different embodiments of the device will now be described with reference to the accompanying figures.

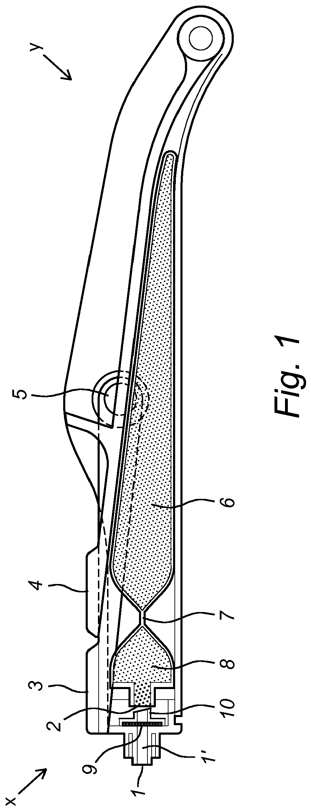

Referring to **Fig. 1****,** a first embodiment of the device is shown. The device comprises a first chamber **8** housing Mesna in solid form and a second chamber **6** housing a buffer having a pH of at least 8.5. The chambers are separated from each other by a disruptable separation means. In this embodiment, the chambers are also attached to each other by the same disruptable separation means which is in this case a disruptable membrane **7.** The first chamber **8** is sealed by a disruptable sealing membrane **2.** The proximal end **X** of the device is provided with an outlet **1** and an outlet tube **1'** for guiding the mixture out of the device. The outlet tube **1'** is movable and its distal end **Y** is suitable to disrupt the sealing membrane **2** of the first chamber **8.** Said distal end of the outlet tube **1'** might have a pointed shape as shown in **Fig. 1** or might be of any other type and/or shape suitable to disrupt the sealing membrane **2.** The device further comprises a microfilter **9** positioned between the outlet **1** of the device and the first chamber **8** which is suitable to be in fluid communication with said outlet **1.** Preferably, the microfilter is integrated with and/or within the outlet tube **1'.** Said microfilter enhances the sterility and prevents contamination of the device and the solution and/or powder contained therein. In a preferred embodiment, said microfilter is made of polyethylene terephtalate, nylon, polyethersulfone or polyamide membrane.

The device shown in **Fig. 1** is further provided with a disruption means for disrupting the separation means, i.e. disruptable membrane **7.** Said disruption means comprises a lever **4,** a trigger **3** and a gear ratchet **5,** preferably a gear with locking ratchet. The trigger **3** is movable from a position in which its proximal end **X** is in contact with the device, called down position, to a position in which the proximal end **X** of the trigger **3** is not in contact with the device, called up position. The lever **4** is in contact with the gear ratchet **5** and also in contact with one of the chambers, preferably with the second chamber **6** which is at the distal end **Y** of the device, more preferably with the device containing the liquid solvent. With this design, the movement of the trigger **3** leads to the rotation of the gear ratchet **5** which in turn moves the lever **4,** thereby applying a pressure on the second chamber **6.** The steps showing the use of the device of **Fig. 1** are illustrated in **Fig. 1A** to **1D****.**

The device is simple to use and offers a rapid system for dissolving the solute in the solvent. The device is also practical as it is suitable to be manually held by the user such as said holding is comparable to a pen holding wherein the index finger is moving the trigger **3** of the device.

The device is provided with control means for the control of fluid volume flowing out of the device. Said control means comprises the trigger **3,** the gear ratchet **5** and the lever **4** which are designed and/or positioned such as a predetermined volume of solution is flowing out of the device with each movement of the trigger **3** from its up position to its down position. In this way, the user can have a control over the amount of fluid flowing out of the device, thereby avoiding any excess of delivered solution volume. In addition, the user will be provided with a control over the time at which the fluid is flowing out of the device. These possibilities are not offered by the devices of the prior art.

In a preferred embodiment, the trigger and/or the lever and/or the gear with locking ratchet and/or the outer walls of the device are made of injectable plastic material. In a preferred embodiment, the separation means and/or the sealing membrane are made of aluminium laminate.

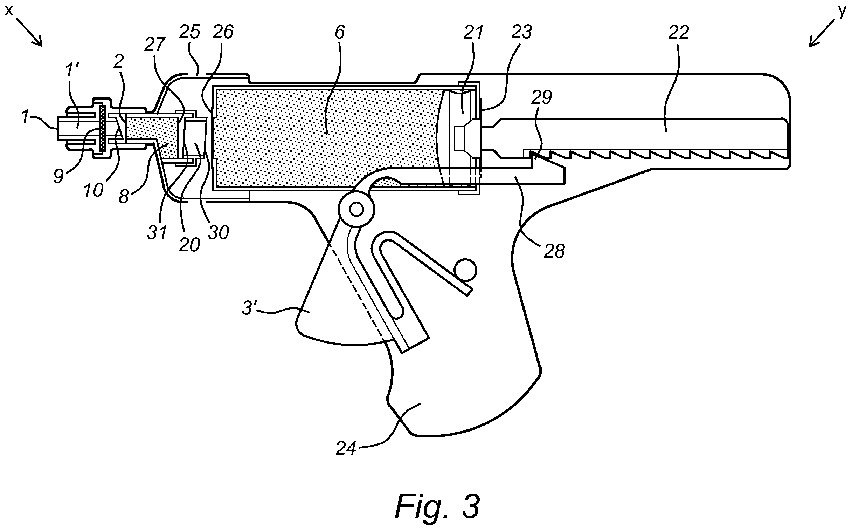

**Fig. 3** shows a second embodiment of the device is shown. The device comprises a first chamber **8** housing Mesna in solid form and a second chamber **6** housing a buffer having a pH of at least 8.5. At least one of the walls of each chamber is at least partially made of a disruptable membrane. The chambers are separated from each other by a separation means which comprises a spatial separation between the first chamber and the second chamber. This means that the chambers are spatially separated from each other. The separation means further comprises the disruptable membranes **26, 27** of said chambers. In this embodiment, at least one of said chambers is movable towards the other chamber which can be movable or non-movable. Preferably said movement is a sliding movement. The first chamber **8** is sealed by a disruptable sealing membrane **2** which is not forming a part of the separation means. The proximal end **X** of the device is provided with an outlet **1** and an outlet tube **1'** for guiding the mixture out of the device. The outlet tube **1'** is movable and its distal end **Y** is suitable to disrupt the sealing membrane **2** of the first chamber **8.** Said distal end of the outlet tube **1'** might have a pointed shape as shown in **Fig. 3** or might be of any other type and/or shape suitable to disrupt the sealing membrane **2.** The device further comprises a microfilter **9** positioned between the outlet **1** of the device and the first chamber **8** which is suitable to be in fluid communication with said outlet **1.** Preferably, the microfilter is integrated with and/or within the outlet tube **1'.** Said microfilter enhances the sterility and prevents contamination of the device and the solution and/or powder contained therein. In a preferred embodiment, said microfilter is made of polyethylene terephtalate, polyethersulfone, nylon or polyamide membrane. The device is also provided with a handle **24** via which the device is hold in a way similar to a pistol hold.

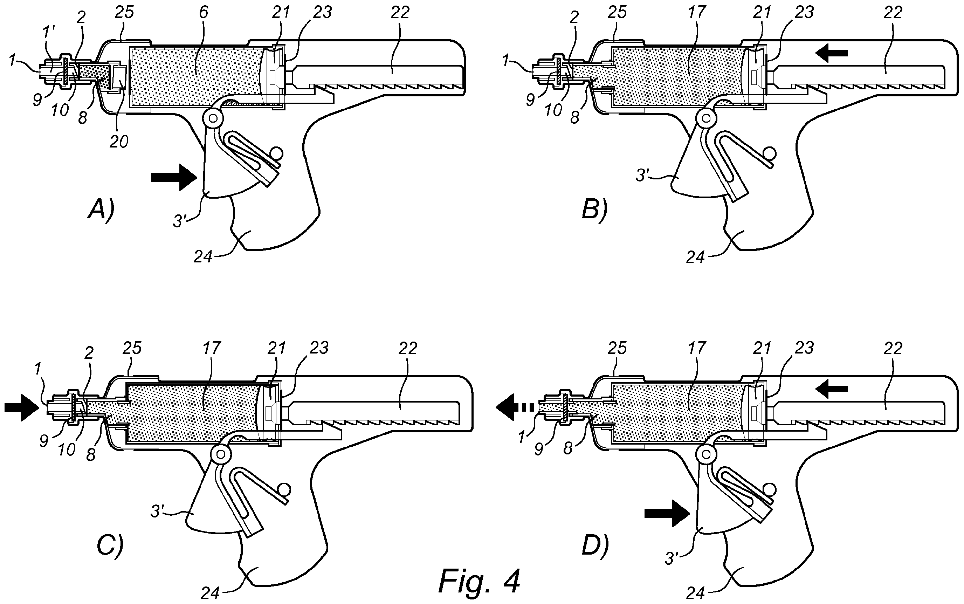

The device shown in **Fig. 3** is further provided with a disruption means for disrupting the separation means. Said disruption means comprises a piercing means **20,** trigger **3',** a linear ratchet **22** and a plunger **21.** The piercing means is positioned between the first chamber **8** and the second chamber **6**; preferably said piercing means **20** is positioned between the disruptable membranes **26, 27** of the chambers which are part of the separation means of the device. Said piercing means **20** is provided with at least two opposed piercing members **30, 31** for piercing and disrupting the disruptable membranes **26, 27** of the first and the second chambers. The piercing means **20** might be fixed to the first chamber **8** as shown in **Fig. 3****.** Alternatively, said piercing means **20** might be fixed to the second chamber **6** or to the walls of both chambers. The trigger **3'** is suitable to be squeezed or pushed towards the handle **24** of the device. Said trigger **3'** is connected to a sliding block **28** having a drive tooth **29** suitable to engage a tooth of the linear ratchet **22** (**Fig. 3**). When the device is not used, the trigger **3'** is in "off position" in which it is not pushed in the handle **24** and the drive tooth **29** is engaging the most proximal tooth of the linear ratchet **22** as shown in **Fig. 3****.** Preferably, the shape of the proximal end **X** of the linear ratchet **22** is form fitting the plunger **21** distal end **Y.** Said plunger **21** is positioned between the linear ratchet **22** and one of the chambers of the device, preferably the second chamber **6** containing the solvent as shown in **Fig. 3****.** The distal end **Y** of the plunger **21** might be provided with a disruptable sealing membrane **23** (**Fig. 3**). The steps showing the use of the device of **Fig. 3** are illustrated in **Fig. 4A** to **4D****.**

The device is simple to use and offers a rapid system for dissolving the solute in the solvent. The device is also practical as it is suitable to be manually held by the user such as said holding is comparable to a pistol holding wherein the index finger is moving the trigger **3'** of the device.

The device is provided with control means for the control of fluid volume flowing out of the device. Said control means comprises the trigger **3',** the sliding block **28** and the linear ratchet **22** which are designed and/or positioned such as a predetermined volume of solution is flowing out of the device with each movement of the drive tooth **29.** By movement of the drive tooth, we refer to the sliding of said drive tooth by which the drive tooth **29** will engage the neighboring teeth of the linear ratchet **22.** In this way, the user can have a control over the amount of fluid flowing out of the device, thereby avoiding any excess of delivered solution volume. In addition, the user will be provided with a control over the time at which the fluid is flowing out of the device. These possibilities are not offered by the devices of the prior art.

Referring to **Fig. 5A****,** another embodiment of the device is shown. The device comprises a first chamber **50** for housing Mesna in solid form; a second chamber **51** for housing a buffer having a pH of at least 8.5 and at least one outlet **55** for delivering the solution. Said outlet is suitable to be in fluid communication with at least one of the chambers. In **Fig. 5A****,** the outlet **55** is suitable to be in fluid communication with the second chamber **51.** Said outlet **55** is covered by a removable cap (not shown) when the device is not used.

The chambers are separated from each other by at least one separation means comprising at least one disruptable separation means (**52, 53** in **Fig. 5B**) and are in fluid communication with each other upon disruption of said disruptable separation means. At least one of the walls of each chamber is at least partially made of a disruptable membrane thereby forming the disruptable separation means. Said disruptable separation means might be two aluminum laminated foils.

**Fig. 5B** shows the details of the separation means. The chambers are separated from each other by a separation means which comprises a spatial separation between the first chamber **50** and the second chamber **51.** This means that the chambers are spatially separated from each other and when the device is not in use, the chambers do not share any common element, such as membranes and/or walls. The separation means further comprises the above mentioned disruptable membranes **52, 53** of said chambers. At least one of said chambers is movable towards the other chamber which can be movable or non-movable. Said movement can be a sliding and/or a rotating movement. By preference, the first chamber **50** is movable towards the second chamber **51** which is non-movable. The first chamber **50** is sealed by a non-disruptable sealing membrane **60** which is not forming a part of the separation means.

The device is further provided with a disruption means **56** for disrupting the disruptable separation means, more in particular for disrupting the disruptable membranes **52** and **53.** Said disruption means comprises at least one piercing means **56.** The piercing means is positioned between the first chamber **50** and the second chamber **51.** Preferably said piercing means **56** is comprised in the separation means and is positioned between the disruptable membranes **52, 53** of the chambers which are part of the separation means of the device. Said piercing means **56** is provided with at least two opposed piercing members for piercing and disrupting the disruptable membranes **52, 53** of the first and the second chambers (**Fig. 5B****).** In a preferred embodiment, the disruptable membranes **52, 53** are made of aluminium laminate.

In a preferred embodiment, the device is provided with at least one air vent **57** for evacuating and/or inserting air from the device. Said air vent is covered by non-disruptable sealing membrane **60** when the device is not used (**Fig. 5B**)**.** Said air vent **57** is preferably provided at the distal end **W** of the device outer wall. **Fig. 5C** shows a cross section view of the device wherein the chambers are pierced by the piercing means **56.**

The chambers of the device might be filled separately within a clean production environment. After filling, the device is assembled with a disruption means **56** inserted between both chambers. The assembled device is placed in a sealed pouch and terminally sterilized by irradiation. The device is stored until use.

Before use, the sterile device is removed from the pouch and the Mesna solution is reconstituted by pushing-in the first chamber **50** against the second chamber **51.** This causes the disruption of the separation means and the dissolution of Mesna powder into the buffer. After mixing and dissolution, which takes from 1 to 60 seconds, the device is ready to be connected to an irrigated surgical instrument such as canulated elevators. The volume of the Mesna formulation is at most 200 ml, preferably at most 150 ml, more preferably at most 100 ml, even more preferably at most 50 ml, most preferably at most 30 ml, thereby making the device more ergonomic.

In use, the user moves the first chamber **50** towards the second chamber **51.** The movement leads to the disruption of the disruptable membranes **52, 53** by the piercing means **56 (****Fig. 5C****).** The content of the first and the second chambers will merge to obtain the Mesna solution. The device can be further agitated or shacked to ensure complete dissolution of Mesna in the buffer. Afterwards, the removable cap is removed thereby uncovering the outlet **55.** The foil tab **60** is then removed for allowing air to replace the volume of the dispensed solution. The outlet of the device can then be used for direct delivery of the Mesna solution to the target or can be connected to any other suitable device such as a dissector **61.** It is to be understood that the air vent **57** and the tab **60** can be replaced by an air permeable membrane.

In a preferred embodiment, the device might be provided with a pressure means **58** for manually applying a pressure on the first chamber walls and/or the second chamber walls thereby delivering the solution to said tissues and/or organs. The pressure means **58** is preferably visible to the user. The pressure means **58** can be a flexible button. Preferably, the second chamber is fitted with the pressure means **58.** Said pressure means **58** is suitable for the application of a manual pressure thereby delivering the solution to said tissues and/or organs. The uncovered air vent **57** admits air when the flexible wall of the chamber returns to its stable position.

In a preferred embodiment, the Mesna solution is delivered in droplets form by applying a manual pressure on pressure means **58.** The control means of the device comprise the pressure means **58.** The droplets have a predetermined volume which is of from 50 to 300 µl, preferably from 60 to 200 µl, more preferably from 70 to 150 µl, most preferably from 80 to 100 µl. In this way the user is capable of hand manipulating the device for directly delivering the Mesna solution from the device to the desired location and in desired volumes. The design, the manufacture and the use of the device are simple thereby saving costs and working time. Furthermore, the device offers a possibility to control the amount of fluid flowing out of the device, thereby avoiding any excess of delivered solution volume. In addition, the user will be provided with a control over the time at which the fluid is flowing out of the device. These possibilities are not offered by the devices of the prior art.

**Fig. 5'** shows a longitudinal cross section of a device which can be used to prepare a mesna solution based on similar principles, except that the surgical instruments are now separated from the cartridge and fluid lines illustrated in **Fig 5D** are not used. The device comprises a first chamber **50** and a second chamber **51** separated by at least one disruptable separation means **52,53.** A disruption means **56** is positioned between the first and the second chamber. The device is provided with a hanger **160** which presence is optional. The device comprises at least one air vent **57** and a cap **163** which closes the outlet of the device and has to be removed prior connecting the device to a second device. To ensure the connection to a second device, a luer activated valve **162** is positioned between the second chamber **51** and the outlet of the device.

As shown in **FIG. 5D****,** the device **11** can be provided with a hanger **160** for hanging it to a pole in a surgery room. The outlet **55** of the device can be connected to a surgical instrument by a sterile tubing **12.** Three types of outlet **55** connections are illustrated in **Fig 5E****:** connection to an open surgery tool with a manual dispenser **13,** connection to an open surgery tool with a remote pump **14** to control dispensing of the Mesna solution, and connection to a laparoscopic or endoscopic instrument operated manually or by surgery robots **15.** In a preferred embodiment, a filter might be inserted in the tubing **12** to avoid contamination with microparticules and bacteria larger than a defined size (usually from 0.2 to 10 microns).

**Fig.** 6 shows another embodiment of the device. Said device comprises a first chamber **101** comprising Mesna in solid form and a second chamber **102** comprising the buffer. The first and the second chambers are separated from each other by at least on disruptable separation means which is represented by the cap **112** of the first chamber and the port **106** on the chamber **102.**

The content of the first chamber **101** is dissolved into the second chamber **102** after disrupting the separation means and fluidly connecting both chambers using a sterile fluid line **107** with pumping the liquid in and out of the first chamber **101.** In this embodiment, the second chamber is provided with a at least one port **106** for fluidly connecting the second chamber with the first chamber. The second chamber is preferably provided with at least one outlet **108** for delivering the Mesna formulation. Said outlet **108** is connectable to a surgical device such as manual instruments or minimally invasive irrigated instruments inserted in trocars or robots. As show in **Fig. 6****,** a peristaltic pump **104,** a foot switch **105** and at least one connection tube **110, 103** might be used depending on the surgical device **109** which is to be connected to the second chamber **102.**

The present invention further provides a process for the preparation of a Mesna formulation. The process comprises the steps of dissolving Mesna in a buffer thereby obtaining the Mesna formulation, wherein the process is devoid of steps in which stabilizers and/or antioxidants are added after dissolving Mesna in the buffer and is characterized in that the buffer pH is at least 8.5. The Mesna is preferably in solid form which is crystalline form or lyophilized form. The buffer is preferably devoid of carbon atoms.

In a preferred embodiment, the dissolution step is performed in a device as provided by any embodiment of the present invention. The process further comprises the steps of providing a device comprising two chambers as described above, disrupting the separation means of the device thereby obtaining the Mesna solution, and delivering the obtained Mesna solution through the outlet of the device to a target location, in particular to tissues and/or organs. In a preferred embodiment, the process comprises the steps of filling Mesna in solid form in a first container and filling the buffer having a pH of at least 8.5 in a second container. Said containers are suitable to be inserted in the device. Each filling step might be performed in aseptic conditions or in non-aseptic conditions followed by a sterilization step. The Mesna container and/or the buffer container might be stored and be later inserted into the device. This allows avoiding aseptic filling of the device chambers which facilitates the production process.

The process according to any embodiment of the invention provides for the controlled delivery of Mesna solution. Said controlled delivery might be performed using control means of the device and/or using an electrically driven mechanical system. Said system can be a syringe driver, a syringe pump or any other system known to the person skilled in the art. Said system is preferably controlled by the practitioner using a pedal connected to the electrically driven mechanical system. This is advantageous as it provides the practitioner with a high hands freedom level required for instance for laparoscopic surgery also called minimal invasive surgery. Indeed, said practitioner will be able to use both hands for operations other than handling the device for delivering Mesna solution to the target location.

The invention further provides a method for weakening and/or organs by delivering a Mesna formulation to tissues and/or organs. The formulation is as described above. The formulation can be delivered to said tissues and/or organs by a device according to any embodiment of the present invention.

The present invention further provides a kit comprising a device as described above and/or a second device selected from the group comprising surgical devices, high pressure pumps, delivery tubes and applicators and/or at least one first container filled with Mesna as described above and/or at least one second container filled with a buffer as described above. The kit further comprises a leaflet provided with user's instructions and/or information on the Mesna and/or the buffer and/or the device of said kit. The kit optionally comprises at least one tubing and at least one dispenser or a tubing compatible with a peristaltic pump.

The present invention further provides for the use of a device and/or a process according to any embodiment of the invention for delivering a formulation comprising Mesna dissolved in a buffer having a pH of at least 8.5, preferably at least 8.7, more preferably at least 8.8, most preferably at least 9 to target tissues and/or organs. The present invention additionally provides for the use of a Mesna formulation for weakening inter-tissues and/or organs adhesion.

It is to be understood that for all the embodiments of the device and/or the method and/or the process of the present invention, the Mesna amount and the buffer volume are selected such that the Mesna concentration of the formulation is of at most 10%, even more preferably at most 8%, most preferably at most 5%. Said Mesna concentration is at least 1%, preferably at least 2%, more preferably at least 3% and most preferably at least 4% or any value comprised between the aforementioned values.

It is to be understood that for all the embodiments of the device and/or the method and/or the process of the present invention, the Mesna in powder contained in the device and/or the buffer is sterilized, preferably by gamma irradiation or X-ray. The irradiation is preferably performed at least at 20 kGy, preferably at least 25 kGy, more preferably at least 30 kGy and at most 50 kGy, preferably at most 45 kGy.

In the embodiments where the powder chamber is physically separated from the liquid chamber, sterilization of the powder can be obtained by ultrafiltration of an aqueous solution followed by aseptic filling and lyophilization into the powder container or vial. Similarly, the buffer solution can be sterilized by classical means before aseptic filling in the liquid container or pouch.

### Examples

### - Buffers

We mixed different buffers with 5% weight/volume Mesna. The pH of the used buffers was at least 8.5, preferably at least 8.7, more preferably at least 8.8, most preferably at least 9. The buffers had different concentrations. The pH of the obtained Mesna formulation was measured. The obtained results are shown in Table 1.

**Table 1: pH of Mesna formulation after mixing Mesna 5% weight/volume with different buffers**

| Buffer composition | Buffer concentration in mM | Product used for pH adjustment | pH before mixing with Mesna | pH after mixing with Mesna |
|---|---|---|---|---|
| Sodium acetate | 12.5 | NaOH | 9.0 | 6.2 |
| Sodium acetate | 25 | NaOH | 9.0 | 6.4 |
| Sodium acetate | 37.5 | NaOH | 9.0 | 6.5 |
| Sodium acetate | 50 | NaOH | 9.0 | 6.6 |
| Sodium bisphosphate | 2.5 | None | 9.0 | 6.8 |
| Sodium bisphosphate | 5 | None | 9.0 | 7.0 |
| Sodium bisphosphate | 10 | None | 9.2 | 7.2 |
| Sodium bisphosphate | 20 | None | 9.3 | 7.3 |
| Sodium bisphosphate | 50 | None | 9.4 | 7.6 |
| Tris | 10 | HCl | 9.0 | 7.6 |
| Tris | 20 | HCl | 9.0 | 7.9 |
| Glycine | 10 | NaOH | 9.0 | 7.0 |
| Glycine | 20 | NaOH | 9.0 | 7.4 |
| Glycine | 50 | NaOH | 9.0 | 7.6 |

The obtained results show that the tested buffers after mixing with 5% Mesna weight/volume provide a Mesna formulation having a pH of from 6 to 8 provided said buffers have a pH of at least 8.5, preferably at least 8.7, more preferably at least 8.8, most preferably at least 9.

### - Mesna sterilization by irradiation

Mesna purity has been evaluated prior and after sterilization using X-rays and gamma irradiation. The purity results are shown in Table 1a below.

**Table 1a: Mesna purity before and after sterilization using X-rays and gamma irradiation**

| | Prior irradiation | After X-ray irradiation | After gamma irradiation |
|---|---|---|---|
| Mesna powder | 89.6 | 89.0 | 88.9 |
| Pure lyophilized Mesna | 89.4 | 89.3 | 89.1 |
| Lyophilized mixture of Mesna and Mannitol | 89.8 | 88.9 | 88.7 |

The results show that the used irradiations have very limited effect on Mesna purity. Indeed the maximal observed purity loss was of about 1%. Therefore Mesna powder or lyophilized Mesna can be sterilized by irradiation.

### - Toxicity of Mesna formulations having different Mesna concentrations

In vitro cytotoxicity of Mesna in human MRC-5 fibroblasts was evaluated. Two experiments were performed. The first one was performed with two doses of Mesna (5% and 10%) and NaCl (7.06 and 3.53%) and the second one was performed with three doses of Mesna (5, 10 and 20%) and the three corresponding doses of NaCl (7.06, 3.53 and 1.76%). All samples were assayed in triplicate. Evaluation of cytotoxicity was evaluated after the following times: T15 min, T30 min, T1h, T2h, T4h, T8h and T24h.

Cells were thawed according to the standard operating procedure No. TEC-005 and subcultured according to the standard operating procedure No. TEC-001. The cell line was cultured in 75 cm²-flasks in appropriated culture medium.

Qualitative evaluation of cytotoxicity: At observation time T15 min, T30 min, T1h, T2h, T4h, T8h and T24h, cells were examined microscopically and the changes in general morphology, vacuolization, detachment, cell lysis and membrane integrity was observed and assessed compared to the non-treated cells. A score was assigned based on observations. The criteria of the scores are specified in table 2 and results are provided in table 3.

**Table 2: Criteria for qualitative evaluation of cytotoxicity by microscopically examination**

| **Score** | **Reactivity** | **Observation** |
|---|---|---|
| 0 | None | Small intracytoplasmic granules, no cell lysis, no viability decreasing. |
| 1 | Light | A maximum of 20% of the cells are round, lightly attached without intracytoplasmic granule or morphological modification. Some lysed cells are observed, a lightly inhibition of growth is observed. |
| 2 | Mild | A maximum of 50% of the cells are round, without intracytoplasmic granule, no important cell lysis, a maximum of 50% of growth inhibition is observed. |
| 3 | Moderate | A maximum of 70% of the cells are round or lysed, cell layers are not totally destroyed but more than 50% of growth inhibition is observed. |
| 4 | Severe | Total or nearly total destruction of cells layers |

No cytotoxicity was observed by microscopic examination of cells incubated in cell culture medium only. Significant cell damages with a maximum of 70% of round or lysed cells and more than 50% of growth inhibition were observed (score 3) as of 15 minutes of exposure to phenol 0.5% positive control (table 3). Overall, there was a clear, concentration dependent, trend of cytotoxicity observed over time exposure for both Mesna and NaCl solutions (table 3). There were no or very little differences between the cytotoxicity of each tested concentration of Mesna and the corresponding NaCl solution at similar osmolarity (table 3). The increase in severity of cytotoxicity being slightly slower in Mesna 20% and 10% and quicker in Mesna 5% compared to their NaCl counterparts. At 30 minutes, effects of Mesna 5% seemed to be more important than Mesna 10 or 20%.

**Table 3: Qualitative evaluation of cytotoxicity by microscopically examination of the MRC-5 cell line treated with Mesna at 20%, 10% or 5%, Phenol at 0.5%, NaCl control at 7.06%, 3.53% or 1.76% or culture medium for 15 min, 30 min, 1h, 2h, 4h, 8h or 24h. The scores indicated are the mean of the two scores. Values are indicated as mean ± SD.**

| | **15min** | **30min** | **1h** | **2h** | **4h** | **8h** | **24h** |
|---|---|---|---|---|---|---|---|
| **Mesna 20%** | 0.0 | 1.0 | 2.0 | 3.0 | 3.0 | 3.0 | 4.0 |
| **NaCl 7.06%** | 0.0±0.0 | 0,5±0,7 | 2,5±0,7 | 3.0±0.0 | 3.0±0.0 | 3.0±0.0 | 4±0 |
| **Mesna 10%** | 0.0±0.0 | 0.0±0.0 | 1.0±0.0 | 3.0±0.0 | 3.0±0.0 | 3.0±0.0 | 4±0 |
| **NaCl 3.53%** | 0.0±0.0 | 0.0±0.0 | 2±1,4 | 3.0±0.0 | 3.0±0.0 | 3.0±0.0 | 4±0 |
| **Mesna 5%** | 0.0±0.0 | 1,5±0,7 | 1,5±0,7 | 3.0±0.0 | 3.0±0.0 | 3.0±0.0 | 4±0 |
| **NaCl 1.76%** | 0.0 | 0.0 | 1.0 | 2.0 | 3.0 | 3.0 | 4.0 |
| **Phenol 0.5%** | 3.0±0.0 | 3.0±0.0 | 3.0±0.0 | 3.0±0.0 | 3.0±0.0 | 3.0±0.0 | 4.0±0.0 |
| **Culture medium** | 0.0±0.0 | 0.0±0.0 | 0.0±0.0 | 0.0±0.0 | 0.0±0.0 | 0.0±0.0 | 0.0±0.0 |

Quantitative evaluation of cytotoxicity MTT assay: The quantitative cytotoxicity was evaluated after T15 min, T30 min, T1h, T2h, T4h, T8h and T24h treatment by viability assay using MTT reagent. The cytotoxicity is defined by a viability of a test substance below 70% in comparison to culture medium. The percentages of cell viability are shown in table 4 and Table 5.

**Table 4: Quantitative evaluation of cytotoxicity of the MRC-5 cell line treated with Mesna at 20%, 10% or 5%, Phenol at 0.5%, NaCl control at 7.06%, 3.53% or 1.76% or culture medium for 15 min, 30 min, 1h, 2h, 4h, 8h or 24h. Results are expressed as percentage of cell viability compared to culture medium (100% of viability).**

| Time in h | Mesna 20% | NaCl 7.06% | Mesna 10% | NaCl 3.53% | Mesna 5% | NaCl 1.76% | Phenol 0.5% |
|---|---|---|---|---|---|---|---|
| 0.25 | -10,2±5,2* | 45.2±11.9* | 62.3±5.9* | 82.7±3.7* | 66±5* | 88.8±1.2* | 10.9±2.3 |
| 0.5 | -1.2±12.8* | 25.3±5.7* | 54.8±7.1 | 55.4±13.7 | 47.6±20.9* | 102.6±3.2* | 13.2±4.6 |
| 1 | 47.2±4.7° | 12.7±4.2 | 20.6±3.7 | 16.3±5.8 | 50.4±8.1* | 95±3.3* | 11.3±2.5 |
| 2 | 11.8±0.6 | 12.3±2.4 | 4.1±2.7 | 12.7±2.6 | 39.7±10.8* | 75.5±13.7* | 13.6±3.6 |
| 4 | 4.6±7.3 | 10.8±1.8 | 3.2±2.4 | 10.5±1.6 | 12±4.7* | 44±0.4* | 10.4±1.7 |
| 8 | 12.1±17.7 | 14.6±6.4 | 3.3±5.2 | 14.4±6.2 | 3.4±2.5* | 23.1±1* | 14.2±5.6 |
| 24 | 18.3±7 | 7.3±0.7 | 1.1±2.8 | 7.2±0.8 | 1.4±3.4 | 8±0.4 | 8.5±1.5 |

°Excluded value (not in compliance with qualitative observations); * significant difference between Mesna and corresponding NaCl (p<0.05).

According to the ISO 10993-5:2009 guidelines, the lower the value of viability (in percentage), the higher is the cytotoxicity of the tested substance. The results of the quantitative evaluation of cytotoxicity confirm the results of the qualitative analysis: in the positive control group phenol 0.5% cytotoxicity (percentage of viability below 70% compared to culture medium) was observed at all time points.

Overall, there was a clear, concentration dependent, trend of cytotoxicity observed over time exposure for both Mesna and NaCl solutions. Globally the cytotoxicity of Mesna 5% is lower than the cytotoxicity of Mesna 10% and 20%, justifying the preference for Mesna low concentrations, i.e 5% and less, compared to high Mesna concentrations, i.e. 10% or 20%, for clinical indications. This is particularly evident for exposure times between 1 and 4 hours (table 5).

There were no or very little differences between the cytotoxicity of each tested concentration of Mesna 20% or 10% and the corresponding NaCl solutions at similar osmolarity (7.06% and 3.53% respectively). The time to reach cytotoxicity (70 % of cell viability compared to culture medium) is shorter in Mesna 20% compared to its NaCl counterpart (table 5).

After 15 minutes, Mesna 5 and 10% are very close to the limit of 70% (66 and 62.3% of viability, respectively). NaCl at 1.76% is not cytotoxic after 2 hours.

**Table 5: Comparison between the various Mesna concentrations**

| **Time** | **Mesna 5%** | **Mesna 10%** | **Mesna 5%** | **Mesna 20%** | **Mesna 10%** | **Mesna 20%** |
|---|---|---|---|---|---|---|
| 15min | 66±5 | 62.3±5.9 | 66±5* | -10,2±5,2* | 62.3±5.9* | -10,2±5,2* |
| 30min | 47.6±20.9 | 54.8±7.1 | 47.6±20.9* | -1.2±12.8* | 54.8±7.1* | -1.2±12.8* |
| 1h | 50.4±8.1* | 20.6±3.7* | 50.4±8.1 | 47.2±4.7° | 20.6±3.7 | 47.2±4.7° |
| 2h | 39.7±10.8* | 4.1±2.7* | 39.7±10.8* | 11.8±0.6* | 4.1±2.7 | 11.8±0.6 |
| 4h | 12±4.7* | 3.2±2.4* | 12±4.7* | 4.6±7.3* | 3.2±2.4 | 4.6±7.3 |
| 8h | 3.4±2.5 | 3.3±5.2 | 3.4±2.5 | 12.1±17.7 | 3.3±5.2 | 12.1±17.7 |
| 24h | 1.4±3.4 | 1.1±2.8 | 1.4±3.4 | 18.3±7 | 1.1±2.8 | 18.3±7 |

| | | | | | | |
|---|---|---|---|---|---|---|
| * significantly different (p<0.05); ° excluded value | | | | | | |

The results of our experiments show that the effects of Mesna are more deleterious for the cells than NaCl at similar osmolarity. This is more pronounced when low dose of both products (5% Mesna and 1.76% NaCl) are used. This is explained by the fact that the impact on disulfur bounds of Mesna is clearly noticeable whereas at higher dose the hypertonicity impact is largely dominant.

When comparing the 5% and 10% concentrations of Mesna, i.e., the concentrations used in most of clinical trials, Mesna 5% appears clearly less cytotoxic than Mesna 10%, especially after long exposure period of time.

### - Buffer effect on bacterial viability and growth

A buffer consisting of a solution of 10 mM Na₂HPO₄ + 75 mM NaCl and having a pH of 9 was tested for its ability to remain contaminated over time. The buffer was contaminated with a 100 bacteria/ml. The bacteria were selected for their resistance to basic pH and their ability to generate significant endotoxin concentration. The contamination was made in triplicate using a mixture of following 3 bacteria: *Escherichia coli, Pseudomonas aeruginosa* and *Vibrio parahaemolyticus.*

The contaminated buffers were incubated for 3 weeks. A negative control being a non-contaminated buffer was also incubated for 3 weeks. The number of viable bacteria was determined just after inoculation (T0), 1 week after inoculation (T1) and 2 weeks after inoculation (T2). Viability of the bacteria was determined by inoculating 100µl from the contaminated buffer on nutritive agar which was incubated at 35°C for maximum 72h. The results are shown in table 6 below.

**Table 6: number of viable bacteria in the 10 mM Na₂HPO₄ + 75 mM NaCl buffer**

| | **T0** | **T1** | **T2** |
|---|---|---|---|
| Buffer inoculated with the bacterial mixture (triplicate average) | 130 CFU/ml | No growth | No growth |
| Negative control buffer | No growth | No growth | No growth |
| Negative control Agar | No growth | No growth | No growth |

The results show that the buffer of the Mesna formulation inhibits bacterial viability and growth.

### - Buffer effect on bacterial endotoxin production

In parallel to the teste performed for determining the bacterial viability in the buffer of the invention, tests for evaluating bacterial endotoxin production level were carried out. The tests were according to the criteria of the method D of the European Pharmacopoeia (edition 8.6), namely the chromogenic kinetic method.

750 ml of 10 mM phosphate buffer containing 75 mM NaCl were introduced in a sterile sealed flask. Six flasks were prepared of which 3 flasks were inoculated with the bacterial mixture described above and 3 flasks were not inoculated with bacteria (used as negative control).

The cap of the sealed flask was disinfected using ethanol, then 150µl of the sample were taken using a 1 ml syringe and a 25G needle and introduced in an "Endosafe" tube.

The inoculated buffer was then diluted 40 times. A 10 times diluted solution was prepared first. To do this, 900µl of water for bacterial endotoxin test (BET) and 100µl of the inoculated buffer were introduced in an Endosafe® tube. The tube was then covered with a parafilm, vortexed for 30 sec and left aside for 5 min. The 10 times diluted solution is ready. For the preparation of the 40 times dilution, 750 µl of water for BET and 250 µl of the 10 times diluted buffer were introduced in an Endosafe® tube. The tube was then covered with a parafilm, vortexed for 30 sec and left aside for 5 min. The parafilm was removed and 25 µl of the solution were introduced in each channel of the cartridge. The cartridge was analyzed using the Endosafe® nexgen-PTSTM. For replicate samples (three replicates), the whole procedure was performed (independent replicates).

The BET tests were performed at the following time points: day 0, day 4, day 11 and day 21 after inoculation. The results are shown in table 7 below.

**Table 7: BET test results for 10 mM Na₂HPO₄ + 75 mM NaCl buffer**

| | **T0** | **T4** | **T11** | **T21** |
|---|---|---|---|---|
| Buffer inoculated with the bacterial mixture (triplicate average) | <0.2 EU/ml | <0.2 EU/ml | <0.219 EU/ml | <0.23 EU/ml |
| Negative control buffer | 0 EU/ml | 0 EU/ml | 0 EU/ml | 0 EU/ml |

Endotoxin level was always <0.025 EU/ml which is the allowed endotoxin limit for injectable products. The tested buffer does not promote bacterial endotoxin production and prevent bacterial growth. Similar results were obtained for a phosphate buffer (10 mM Na2HPO4) only (i.e. without 75 mM NaCl).

## Claims

1. A device for delivering a Mesna formulation to tissues and/or organs, comprising a first chamber comprising Mesna in solid form, a second chamber comprising a buffer and at least one outlet for delivering the Mesna (sodium 2-mercaptoethanesulfonate) formulation, said outlet is in fluid communication with at least one of the chambers; said chambers are separated from each other by at least one disruptable separation means and are in fluid communication with each other upon disruption of said separation means thereby forming the Mesna formulation, **characterized in that** the pH of the buffer comprised in the second chamber is at least 8.5, wherein the buffer is devoid of carbon atoms.

2. The device according to any of claim 1 wherein the buffer comprises phosphate such that the ratio phosphate to Mesna is at least 1/500 and at most 1/2.

3. The device according to claim 1 or 2 wherein the concentration of the Mesna formulation is at most 10%, preferably at most 5%.

4. The device according to any of claims 1-3 wherein the buffer comprises between 0% and 0.9 % NaCl, preferably between 0.2% and 0.6% of NaCl.

5. The device according to any of claim 1-4 comprising at least one control means which provides for the control of the solution volume flowing out of the device.

6. The device according to any claims 1-5, wherein the outlet is connectable to a second device selected from the group comprising surgical devices, high pressure pumps, delivery tubes, applicators, minimally invasive surgery systems and low pressure pump.

7. The device according to any of claims 1-6 wherein Mesna comprised in the first chamber is in crystalline form or lyophilized form.

8. A process for the preparation of a Mesna formulation, comprising the steps of dissolving Mesna in solid form in a buffer thereby obtaining the Mesna formulation, wherein the process is devoid of steps in which stabilizers and/or antioxidants are added after dissolving Mesna in the buffer and is **characterized in that** the buffer pH is at least 8.5, wherein the buffer is devoid of carbon atoms.

9. The process according to claim 8 wherein the Mesna is in crystalline form or lyophilized form.

10. The process according to claim 8 or 9 wherein the steps of dissolving Mesna in a buffer is conducted in a device as described in any of claims 1-8.

11. The process according to any of claims 8-10 wherein the concentration of the prepared Mesna formulation is at most 10%, preferably at most 5%.

## Patentansprüche

1. Vorrichtung zum Abgeben einer Mesnaformulierung an Gewebe und/oder Organe, eine erste Kammer, die Mesna in fester Form umfasst, eine zweite Kammer, die einen Puffer umfasst, und mindestens einen Auslass zum Abgeben der Mesna(2-Mercaptoethansulfonat-Natrium)-formulierung umfassend, wobei der Auslass in Fluidverbindung mit mindestens einer der Kammern steht, wobei die Kammern durch mindestens ein zerreißbares Trennmittel voneinander getrennt sind und nach dem Zerreißen des Trennmittels in Fluidverbindung miteinander stehen, wodurch die Mesnaformulierung gebildet wird, **dadurch gekennzeichnet, dass** der pH-Wert des Puffers, der in der zweiten Kammer enthalten ist, mindestens 8,5 beträgt, wobei der Puffer frei von Kohlenstoffatomen ist.

2. Vorrichtung nach einem von Anspruch 1, wobei der Puffer Phosphat umfasst, so dass das Verhältnis von Phosphat zu Mesna mindestens 1:500 und höchstens 1:2 beträgt.

3. Vorrichtung nach Anspruch 1 oder 2, wobei die Konzentration der Mesnaformulierung höchstens 10 % beträgt, vorzugsweise höchstens 5%.

4. Vorrichtung nach einem der Ansprüche 1 bis 3, wobei der Puffer zwischen 0 % und 0,9 % NaCl umfasst, vorzugsweise zwischen 0,2 % und 0,6 % NaCl.

5. Vorrichtung nach einem der Ansprüche 1 bis 4, mindestens ein Steuermittel umfassend, das für die Steuerung des Lösungsvolumens sorgt, das aus der Vorrichtung strömt.

6. Vorrichtung nach einem der Ansprüche 1 bis 5, wobei der Auslass an eine zweite Vorrichtung anschließbar ist, die aus der Gruppe ausgewählt ist, die chirurgische Vorrichtungen, Hochdruckpumpen, Abgaberöhrchen, Applikatoren, minimalinvasive Operationssysteme und Niederdruckpumpen umfasst.

7. Vorrichtung nach einem der Ansprüche 1 bis 6, wobei Mesna, das in der ersten Kammer enthalten ist, in kristalliner Form oder lyophilisierter Form vorliegt.

8. Verfahren zur Herstellung einer Mesnaformulierung, den Schritt des Auflösens von Mesna in fester Form in einem Puffer umfassend, wodurch die Mesnaformulierung erzielt wird, wobei das Verfahren frei von Schritten ist, in denen nach dem Auflösen des Mesna in dem Puffer Stabilisatoren und/oder Antioxidantien zugesetzt werden, und **dadurch gekennzeichnet, dass** der pH-Wert des Puffers mindestens 8,5 beträgt, wobei der Puffer frei von Kohlenstoffatomen ist.

9. Verfahren nach Anspruch 8, wobei das Mesna in kristalliner Form oder lyophilisierter Form vorliegt.

10. Verfahren nach Anspruch 8 oder 9, wobei der Schritt des Auflösens von Mesna in dem Puffer in einer Vorrichtung wie in einem der Ansprüche 1 bis 8 beschrieben ausgeführt wird.

11. Verfahren nach einem der Ansprüche 8 bis 10, wobei die Konzentration der hergestellten Mesnaformulierung höchstens 10 % beträgt, vorzugsweise höchstens 5%.

## Revendications

1. Dispositif pour délivrer une formulation de Mesna à des tissus et/ou des organes, comprenant une première chambre comprenant du Mesna sous forme solide, une seconde chambre comprenant un tampon et au moins un orifice de sortie pour délivrer la formulation de Mesna (sodium 2-mercaptoéthanesulfonate), ledit orifice de sortie est en communication fluidique avec au moins l'une des chambres ; lesdites chambres sont séparées l'une de l'autre par au moins un moyen de séparation qui peut être rompu et sont en communication fluidique l'une avec l'autre lors de la rupture dudit moyen de séparation, formant ainsi la formulation de Mesna, **caractérisé en ce que** le pH du tampon compris dans la seconde chambre est d'au moins 8,5, dans lequel le tampon est exempt d'atomes de carbone.

2. Dispositif selon l'une quelconque des revendications 1 dans lequel le tampon comprend du phosphate de telle sorte que le rapport phosphate/Mesna est d'au moins 1/500 et d'au plus 1/2.

3. Dispositif selon la revendication 1 ou 2, dans lequel la concentration de la formulation de Mesna est d'au plus 10 %, de préférence d'au plus 5 %.

4. Dispositif selon l'une quelconque des revendications 1 à 3, dans lequel le tampon comprend entre 0 % et 0,9 % de NaCl, de préférence entre 0,2 % et 0,6 % de NaCl.

5. Dispositif selon l'une quelconque des revendications 1 à 4, comprenant au moins un moyen de commande qui assure le contrôle du volume de solution qui sort de l'appareil.

6. Dispositif selon l'une quelconque des revendications 1 à 5, dans lequel l'orifice de sortie peut être connecté à un deuxième dispositif choisi dans le groupe comprenant des dispositifs chirurgicaux, des pompes à haute pression, des tubes d'alimentation, des applicateurs, des systèmes de chirurgie mini-invasive et une pompe à basse pression.

7. Dispositif selon l'une quelconque des revendications 1 à 6, dans lequel le Mesna compris dans la première chambre est sous forme cristalline ou sous forme lyophilisée.

8. Procédé pour la préparation d'une formulation de Mesna, comprenant les étapes consistant à dissoudre du Mesna sous forme solide dans un tampon de manière à obtenir la formulation de Mesna, dans lequel le procédé est dépourvu d'étapes dans lesquelles des stabilisants et/ou des antioxydants sont ajoutés après la dissolution du Mesna dans le tampon, et est **caractérisé en ce que** le pH du tampon est d'au moins 8,5, dans lequel le tampon est exempt d'atomes de carbone.

9. Procédé selon la revendication 8, dans lequel le Mesna est sous forme cristalline ou sous forme lyophilisée.

10. Procédé selon la revendication 8 ou 9, dans lequel les étapes de dissolution du Mesna dans un tampon sont effectuées dans un dispositif tel que décrit dans l'une quelconque des revendications 1 à 8.

11. Procédé selon l'une quelconque des revendications 8 à 10, dans lequel la concentration de la formulation de Mesna préparée est d'au plus 10 %, de préférence d'au plus 5 %.
